# EUROPEAN PATENT APPLICATION

(11) **EP 4 578 475 A1**
(43) Date of publication of application: **02.07.2025**
(21) Application number: 24152721.7
(22) Date of filing: 18.01.2024
(51) Int. Cl.: A61M 5/142, A61M 5/168, A61M 5/36

(54) **DRUG DELIVERY DEVICE**

(30) Priority: 29.12.2023 US 202318399720
(71) Applicant: Altek Biotechnology Corporation, 300 Hsinchu City (TW)
(72) Inventor: Ku, Hao-Kuang, 300 Hsinchu City (TW); Chu, Yi, 300 Hsinchu City (TW)
(74) Representative: Straus, Alexander

(57) **Abstract**

A drug delivery device (10) includes a drug container (12), a peristaltic pump (14), an orientation sensor (16), an alarm unit and an operation processor (22). The peristaltic pump (14) is adapted to deliver a therapeutic substance of the drug container (12) into a subject. The orientation sensor (16) is adapted to sense orientation (V) of the drug container (12) relative to gravity (G). The operation processor (22) is adapted to acquire a first angle boundary (IAB) and a second angle boundary (OAB) corresponding to the drug container (12), and further to maintain delivery of the peristaltic pump (14) and actuate the alarm unit in response to the orientation (V) greater than the first angle boundary (IAB) but smaller than the second angle boundary (OAB), and further to pause the delivery of the peristaltic pump (14) in response to the orientation (V) greater than the second angle boundary (OAB).

## Description

### Field of the Invention

The present invention relates to a drug delivery device according to the pre-characterizing clauses of claims 1 and 9.

### Background of the Invention

A glass vial sealed by a rubber stopper is the most widely used drug container for filling pharmaceutical therapeutic substances. To access the therapeutic substance inside a vial, a needle is pierced through the rubber stopper and draws the therapeutic substance from the vial. The drawn therapeutic substance is then injected into human body either manually or by using a device with automated functions. In the case of using a drug delivery device that is based on a vial as the source of a therapeutic substance, air can be inadvertently drawn into the fluid path of a device and then get injected into the body of a human subject. Such inadvertent drawing of air can be caused by over-tilting of a vial as a result of undesired device positioning or human posture, especially when the device containing a vial is worn on the body as a wearable injector. Consequently, injecting excessive air into the body may cause harm to heath. Therefore, a drug delivery device capable of automatically controlling the delivery of therapeutic substance by detecting the orientation of a drug container is an important design consideration for a safe and reliable delivery of a therapeutic substance from a non-collapsible container such as a vial.

### Summary of the Invention

This in mind, the present invention aims at providing a drug delivery device for solving above drawbacks.

This is achieved by a drug delivery device according to claims 1 and 9. The dependent claims pertain to corresponding further developments and improvements.

As will be seen more clearly from the detailed description following below, the claimed drug delivery device includes a drug container, a peristaltic pump, a drive system, an orientation sensor and an operation processor. The peristaltic pump is engaged with the drug container and adapted to deliver a therapeutic substance from the drug container to a subject. The drive system is adapted to drive the peristaltic pump to move the therapeutic substance from the drug container. The orientation sensor is adapted to detect an orientation of the drug container relative to gravity. The operation processor acquires a pre-set program, in response to delivered volumes of the therapeutic substance and the orientation of the drug container, and is adapted to provide an output signal to control delivery of the therapeutic substance.

### Brief Description of the Drawings

In the following, the invention is further illustrated by way of example, taking reference to the accompanying drawings. Thereof:
FIG. 1 is a structural diagram of a drug delivery device according to an embodiment of the present invention,
FIG. 2 is a structural diagram of the drug delivery device in other situation according to the embodiment of the present invention,
FIG. 3 is a curve diagram of a first angle boundary and a second angle boundary according to the embodiment of the present invention,
FIG. 4 is a curve diagram of the first angle boundary and the second angle boundary according to another embodiment of the present invention,
FIG. 5 is a diagram of an alarm system according to a first embodiment of the present invention,
FIG. 6 is a diagram of the alarm system according to a second embodiment of the present invention,
FIG. 7 is a diagram of the alarm system according to a third embodiment of the present invention, and
FIG. 8 is a diagram of the alarm system according to a fourth embodiment of the present invention.

### Detailed Description

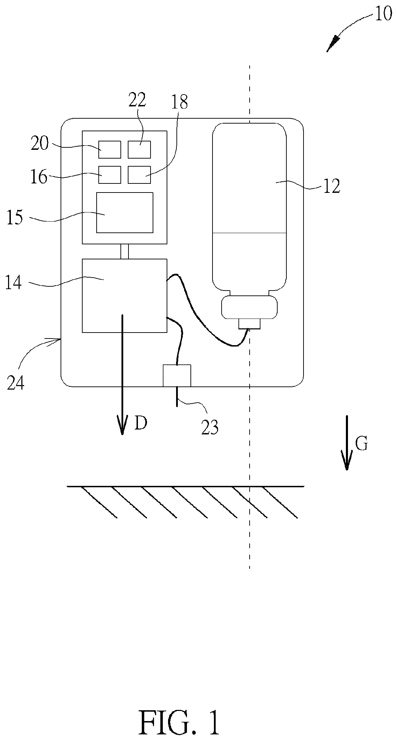

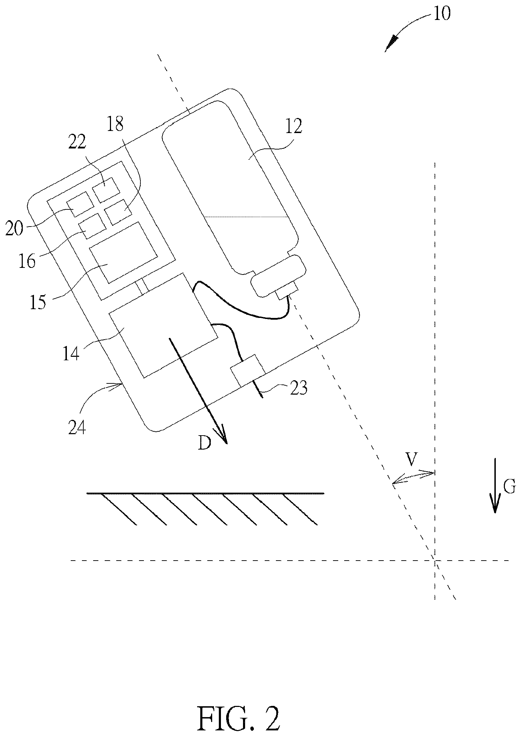

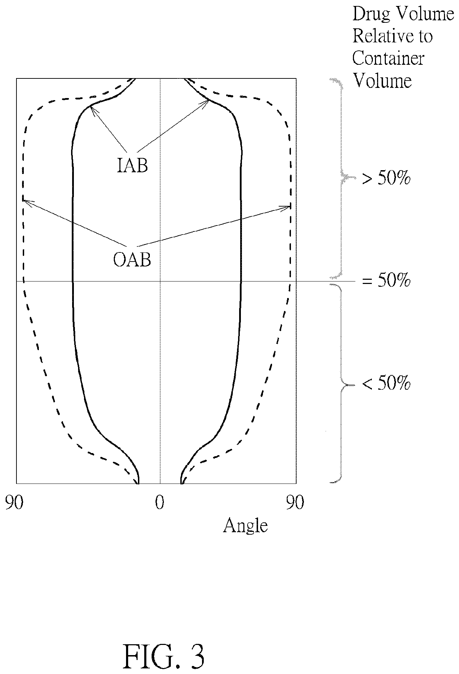

Please refer to FIG. 1 to FIG. 3. FIG. 1 is a structural diagram of a drug delivery device 10 according to an embodiment of the present invention. FIG. 2 is a structural diagram of the drug delivery device 10 in other situation according to the embodiment of the present invention. FIG. 3 is a curve diagram of a first angle boundary IAB and a second angle boundary OAB according to the embodiment of the present invention. The drug delivery device 10 can include a drug container 12, a peristaltic pump 14, a drive system 15, an orientation sensor 16, an alarm system 18, a memory unit 20 and an operation processor 22. The drug container 12 is used to accommodate a therapeutic substance. The peristaltic pump 14 can be connected to the drug container 12 for delivering the therapeutic substance from the drug container 12 directly to a subject via a needle 23 without any pump chamber. In the embodiment, the drug container 12 can preferably be a vial made by hard materials, and the peristaltic pump 14 takes out the therapeutic substance from the vial by suction.

The drive system 15 can be a motor, a micro electromechanical structure or any component that can provide rotational motion, and can be used to drive the peristaltic pump 14 to move the therapeutic substance from the drug container 12. The orientation sensor 16 can be used to detect orientation V of the drug container 12 relative to the gravity G. Generally, the orientation sensor 16 can acquire an angle difference between a longitudinal direction D of the drug container 12 and a direction of the gravity G for setting as the orientation V; however, the present invention may utilize other reference line of the drug container 12 to define the orientation V relative to the gravity G. The alarm system 18 can output a tactile reminder, a visual reminder and/or an audible reminder, which depends on a design demand. The alarm system 18 may include a single or a plurality of alarm units, which are adapted to provide an interface output to output an alarm signal or a combined alarm signal, for informing users the delivery status of the therapeutic substance. A property of the alarm unit is designed in accordance with the tactile reminder, the visual reminder and/or the audible reminder that are output by the alarm system 18, and a detailed description is illustrated later.

The memory unit 20 can store the first angle boundary IAB and the second angle boundary OAB relevant to a specific parameter of the drug container 12. Storage information of the memory unit 20 is not limited to the foresaid embodiment, and depends on the design demand. The specific parameter can be delivered volumes of the therapeutic substance inside the drug container 12 and/or time progression of the delivery of the therapeutic substance. The drug delivery device 10 can analyze an operation parameter of the drive system 15 (such as a number of turns that an element of the drive system 15 is rotated) or any available parameter of other elements to decide the delivered volumes of the therapeutic substance inside the drug container 12; variation of deciding the delivered volumes can depend on the design demand. In addition, the residual volumes in the drug container 12 can be equal to a difference between the total initial volumes of the therapeutic substance inside the drug container 12 and the delivered volumes of the therapeutic substance, so that the specific parameter may be expressed in various ways as the delivered volumes of therapeutic substance or the residual volumes in the drug container 12. The drug delivery device 10 may optionally include a timer used to count the time progression of the delivery of the therapeutic substance. The foresaid specific parameter can be a vial feature or a preset barcode of the drug container 12. The drug delivery device 10 can read and identify the specific parameter of the drug container 12, and search the corresponding first angle boundary IAB and the corresponding second angle boundary OAB from a database of the memory unit 20.

The operation processor 22 can be electrically connected to the peristaltic pump 14, the orientation sensor 16, the alarm system 18 and the memory unit 20. The operation processor 22 may acquire a pre-set program from the memory unit 20 that includes the specific parameter, and the specific parameter includes, but is not limited to, the delivered volumes of the therapeutic substance inside the drug container 12 and/or the time progression of the delivery of the therapeutic substance. The operation processor 22 can acquire the first angle boundary IAB and the second angle boundary OAB relevant to the drug container 12 from the memory unit 20, and decide whether to actuate the peristaltic pump 14 and/or the alarm system 18 in accordance with a comparison result of the orientation V to the first angle boundary IAB and the second angle boundary OAB. The drug delivery device 10 can further include a protective case 24 used to accommodate the drug container 12, the peristaltic pump 14, the orientation sensor 16, the memory unit 20 and the operation processor 22 for protection. The protective case 24 can be designed as one case, or multiple cases assembled together as a single structure. Variation of the protective case 24 can depend on a design demand.

As mentioned above, the alarm system 18 can provide the tactile reminder, the visual reminder and/or the audible reminder, and may be disposed inside the protective case 24 or on an outer surface of the protective case 24 in accordance with an actual application. For example, the alarm system 18 which outputs the visual reminder can be disposed on the outer surface of the protective case 24, so the user can directly see the visual reminder displayed by or on the visual reminder. The alarm system 18 which outputs the audible reminder and/or the tactile reminder can be optionally disposed inside or on the outer surface of the protective case 24; the alarm system 18 can be set on any position that the user can clearly hear voice or hear vibration.

The horizontal axis in FIG. 3 is defined as an angle value, and the vertical axis is FIG. 3 is defined as a volume ratio of the therapeutic substance relative to the drug container 12. Top ends of corresponding curves of the first angle boundary IAB and the second angle boundary OAB can be interpreted as a delivery initiation angle, and bottom ends of the corresponding curves of the first angle boundary IAB and the second angle boundary OAB can be interpreted as a delivery ending angle. In the embodiment, the delivery initiation angle and the delivery ending angle of each of the first angle boundary IAB and the second angle boundary OAB can be designed to have high similarity, which means an angle difference between the delivery initiation angle and the delivery ending angle of any of the first angle boundary IAB and the second angle boundary OAB can be smaller than a predefined threshold; the predefined threshold can be set due to an allowable error of the present invention, such as ten degrees, and a detailed description is omitted herein for simplicity.

In the preferred embodiment, the delivery initiation angle of each boundary of the first angle boundary IAB and the second angle boundary OAB can be smaller than the delivery ending angle of the same boundary of the first angle boundary IAB and the second angle boundary OAB, which depends on the design demand.

In the operation process, the operation processor 22 can set the corresponding first angle boundary IAB and the corresponding second angle boundary OAB in accordance with the specific parameter of the drug container 12. For example, the protective case 24 may be designed to accommodate the drug container 12 with the specific vial feature, and the operation processor 22 can read the first angle boundary IAB and the second angle boundary OAB that are relevant to the specific vial feature directly from the memory unit 20; besides, the protective case 24 may be designed to accommodate the drug container 12 with various types, and the operation processor 22 can identify the preset barcode printed on the drug container 12, or the user can manually input the vial feature of the drug container 12 via an operation interface, so the operation processor 22 can search the suitable first angle boundary IAB and the suitable second angle boundary OAB within the database of the memory unit 20.

As shown in FIG. 3, a range contained by the corresponding curves of the first angle boundary IAB can be defined as a safe zone; a range outside the corresponding curves of the first angle boundary IAB but contained by the corresponding curves of the second angle boundary OAB can be further defined as a danger zone; a range outside the corresponding curves of the second angle boundary OAB can be defined as a pause zone. The orientation sensor 16 can sense the orientation V of the drug container 12 relative to the gravity G at any time. The operation processor 22 can acquire the pre-set program to compare the orientation V with the first angle boundary IAB and/or the second angle boundary OAB in every predefined period of time (such as several seconds or other time units).

The orientation V can be interpreted as staying in the safe zone when the orientation V is smaller than or equal to the first angle boundary IAB, and delivery of the peristaltic pump 14 can be maintained and the alarm system 18 is not actuated; the orientation V can be interpreted as staying in the danger zone when the orientation V is greater than the first angle boundary IAB but smaller than or equal to the second angle boundary OAB, and the delivery of the peristaltic pump 14 can be maintained and the alarm system 18 is actuated; the orientation V can be interpreted as staying in the pause zone when the orientation V is greater than the second angle boundary OAB, and the delivery of the peristaltic pump 14 can be paused and the alarm system 18 can be optionally shut down or actuated. It should be mentioned that the alarm system 18 is an optional element, which means the drug delivery device 10 may exclude the alarm system 18, and the operation processor 22 can only maintain or pause the delivery of the peristaltic pump 14 in accordance with a comparison result of the orientation V to the first angle boundary IAB and/or the second angle boundary OAB; the alarm system 18 is removed or shut down.

Therefore, the orientation V being smaller than or equal to the first angle boundary IAB can indicate that the drug delivery device 10 is set in an upright position, so the delivery of the peristaltic pump 14 can be maintained. The orientation V being greater than the first angle boundary IAB but smaller than or equal to the second angle boundary OAB can indicate that the drug delivery device 10 is slightly tilted within the allowable error, so the delivery of the peristaltic pump 14 can be maintained, and the alarm system 18 can be actuated to remind the user to not further tilt the drug delivery device 10 or to calibrate the drug delivery device 10 back to the upright position. The orientation V being greater than the second angle boundary OAB can indicate that the drug delivery device 10 is excessively tilted and the peristaltic pump 14 may inject gaseous matter from the drug container 12 into the subject, so the delivery of the peristaltic pump 14 is immediately paused.

As shown in FIG. 3, when a large amount of the therapeutic substance is stored in the drug container 12, the first angle boundary IAB and the second angle boundary OAB can be close to ninety degrees, and the drug container 12 can be put in the horizontal state; when the therapeutic substance in the drug container 12 is gradually decreased, the allowable angle of the first angle boundary IAB and the second angle boundary OAB are rapidly reduced, and the drug container 12 has to be set in the upright position for avoiding injection of air .

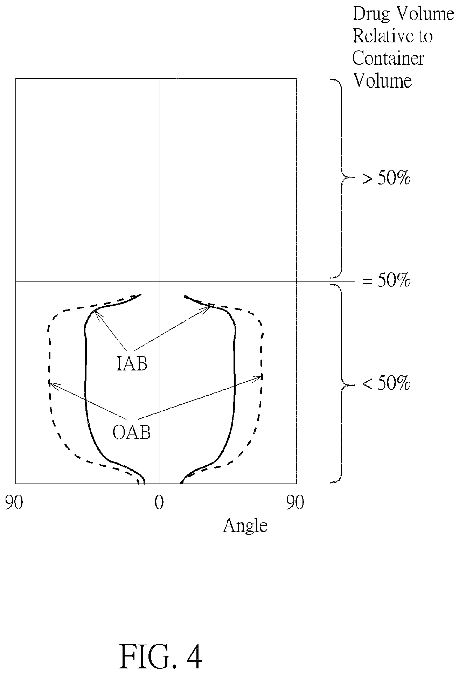

Please refer to FIG. 3 and FIG. 4. FIG. 4 is a curve diagram of the first angle boundary IAB and the second angle boundary OAB according to another embodiment of the present invention. The drug container 12 that is fully filled with the therapeutic substance can correspond to the first angle boundary IAB and the second angle boundary OAB shown in FIG. 3. In other possible situation, the drug container 12 may be filled with a small amount of the therapeutic substance, and related curves of the first angle boundary IAB and the second angle boundary OAB can be shown in FIG. 4; the ranges of the safe zone and the danger zone shown in FIG. 4 can be reduced and smaller than the embodiment shown in FIG. 3 to avoid the injection of air. Thus, the operation processor 22 in this embodiment can acquire a total initial volume of the therapeutic substance inside the drug container 12, and then decide the corresponding first angle boundary IAB and the corresponding second angle boundary OAB in accordance with the total initial volume of the therapeutic substance.

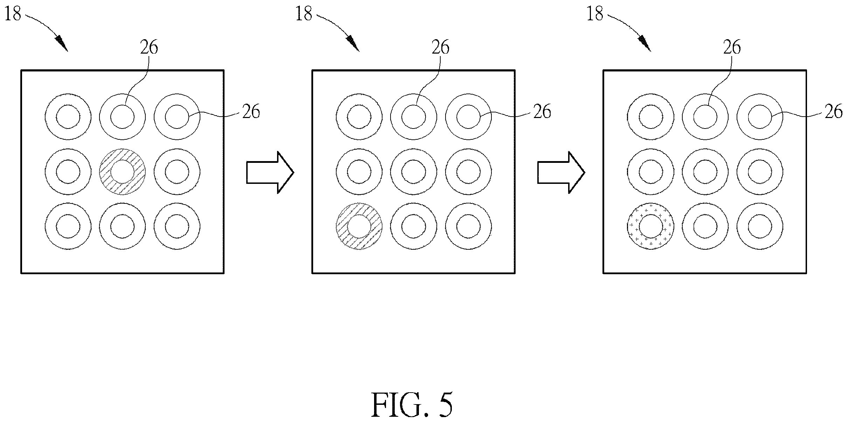

Please refer to FIG. 5. FIG. 5 is a diagram of the alarm system 18 according to a first embodiment of the present invention. The alarm system 18 can include a plurality of lighting units 26 arranged as a matrix. When the orientation V is smaller than or equal to the first angle boundary IAB, a central lighting unit 26 of the plurality of lighting units 26 can be actuated to emit a first color beam, such as green light, and the orientation V of the drug container 12 is in the safe zone. When the orientation V is greater than the first angle boundary IAB but smaller than or equal to the second angle boundary OAB, at least one outer lighting unit 26 of the plurality of lighting units 26 can be actuated to emit the first color beam (or other color beams different from the first color beam), so the orientation V of the drug container 12 is in the danger zone, and a position of the outer lighting unit 26 within the plurality of lighting units 26 can correspond to a tilted direction of the drug container 12 (which means the orientation V). When the orientation V is greater than the second angle boundary OAB, the foresaid outer lighting unit 26 can be actuated to emit a second color beam different from the first color beam, such as red light, and the orientation V of the drug container 12 is in the pause zone. It should be mentioned that a shape of the lighting unit 26 is not limited to circular light sources arranged as the matrix, and can be designed as a strip light source arranged into other geometric patterns; variation of the lighting unit 26 can depend on the design demand.

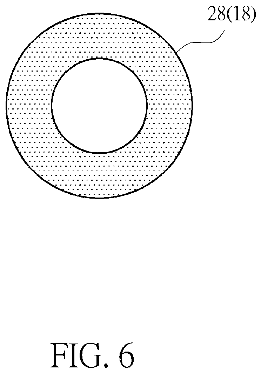

Please refer to FIG. 6. FIG. 6 is a diagram of the alarm system 18 according to a second embodiment of the present invention. The alarm system 18 in the second embodiment can include one lighting unit 28, and color of an illumination beam emitted by the lighting unit 28 can be changed in accordance with the orientation V varied relative to the first angle boundary IAB and/or the second angle boundary OAB. For example, when the orientation V is in the safe zone, the illumination beam of the lighting unit 28 can be the green light; when the orientation V is in the danger zone, the illumination beam of the lighting unit 28 can be yellow light; when the orientation V is in the pause zone, the illumination beam of the lighting unit 28 can be the red light. Color change of the illumination beam emitted by the single lighting unit 28 is not limited to the foresaid embodiment, and depends on the design demand.

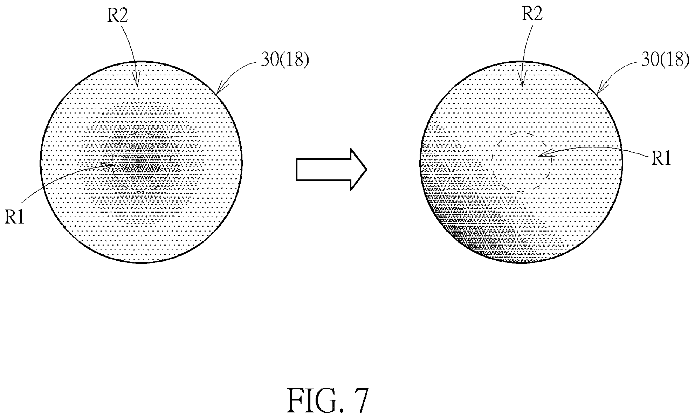

Please refer to FIG. 7. FIG. 7 is a diagram of the alarm system 18 according to a third embodiment of the present invention. The alarm system 18 in the third embodiment can include a display panel 30. When the orientation V is smaller than or equal to the first angle boundary IAB, a central region R1 of the display panel 30 can be actuated to display a specific color block, such as a green block; when the orientation V is greater than the first angle boundary IAB but smaller than or equal to the second angle boundary OAB, a side region R2 of the display panel 30 can be actuated to display the specific color block, and a position of the specific color block inside the display panel 30 can correspond to the tilted direction of the drug container 12 (which means the orientation V); when the orientation V is greater than the second angle boundary OAB, the side region R2 of the display panel 30 can be actuated to change the color of the specific color block, such as a red block. Shapes of the central region R1 and the side region R2 and color change of the specific color block are not limited to the foresaid embodiment, and depend on the design demand.

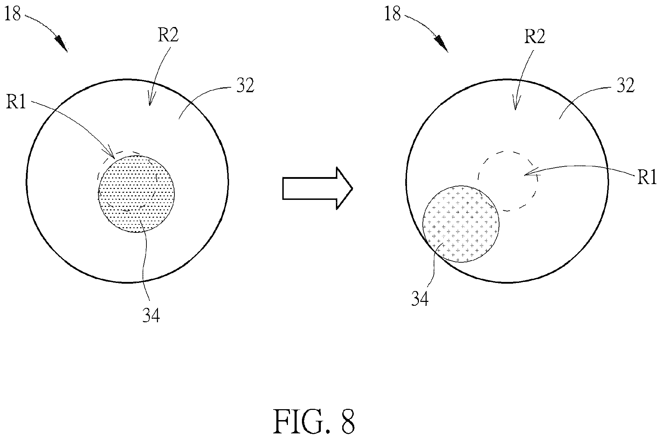

Please refer to FIG. 8. FIG. 8 is a diagram of the alarm system 18 according to a fourth embodiment of the present invention. The alarm system 18 in the fourth embodiment can include a base 32 and a movable element 34. In this embodiment, the base 32 can contain liquid, and the movable element 34 is a suspended unit disposed inside the base 32, such as a bubble or a plastic member. When the orientation V is smaller than or equal to the first angle boundary IAB, the movable element 34 can be located at the central region R1 of the base 32; when the orientation V is greater than the first angle boundary IAB but smaller than or equal to the second angle boundary OAB, the movable element 34 can be located at the side region R2 of the base 32.

In another possible variation, the base 32 can be the display panel, and the movable element 34 can be a specific pattern displayed on the display panel. When the orientation V is smaller than or equal to the first angle boundary IAB, the movable element 34 can be located at the central region R1 of the base 32 and display as the specific green pattern. When the orientation V is greater than the first angle boundary IAB but smaller than or equal to the second angle boundary OAB, the movable element 34 can be located at the side region R2 of the base 32 and display as the specific green pattern or the specific yellow pattern. When the orientation V is greater than the second angle boundary OAB, the movable element 34 can be stayed at the side region R2 of the base 32 and display as the specific red pattern.

Moreover, the alarm system 18 can further include a speaker and/or a piezoelectric element, which is electrically connected to the operation processor 22 and not shown in the figures. The operation processor 22 can drive the speaker to output an audio signal, or drive the piezoelectric element to generate vibration amplitude in accordance with the orientation V varied relative to the first angle boundary IAB and/or the second angle boundary OAB. For example, when the orientation V is smaller than or equal to the first angle boundary IAB, the speaker may not output the audio signal or output the week beep, and the piezoelectric element may be not vibrated or generate the weak vibration amplitude. When the orientation V is greater than the first angle boundary IAB but smaller than or equal to the second angle boundary OAB, the speaker may not output the audio signal or output the medium volume beep, and the piezoelectric element may be not vibrated or generate the medium intensity vibration amplitude. When the orientation V is greater than the second angle boundary OAB, the speaker can output the harsh beep and the piezoelectric element can generate the high intensity vibration amplitude.

In other possible embodiment, the alarm system 18 may integrate the above-mentioned types to simultaneously provide two or three types of the tactile reminder, the visual reminder and the audible reminder. In addition, the drug delivery device 10 can further include the operation interface, and the user can utilize the operation interface to manually select or switch the alarm system 18 to output the tactile reminder, the visual reminder and/or the audible reminder. Application of the alarm system 18 and the operation interface can depend on the actual demand.

In conclusion, the drug delivery device of the present invention can be hung around the user's neck, tied to the arm or the chest, attached to the abdomen or thigh, or placed in the backpack or the waist bag; placement of the drug delivery device is not limited to the foresaid position or any specific position, and can be applied for a wide range of application. The drug delivery device can utilize the orientation sensor to sense the orientation of the drug container relative to the gravity, and the peristaltic pump and the alarm system can immediately respond to the variation of the orientation to ensure the therapeutic substance can be injected into the user accurately and safely within the predefined angle boundary, for enhancing operation safety and providing enhanced user experience.

## Claims

1. A drug delivery device (10), the drug delivery device (10) **characterized by**:
a drug container (12);
a peristaltic pump (14) engaged with the drug container (12) and adapted to deliver a therapeutic substance from the drug container (12) to a subject;
a drive system (15) adapted to drive the peristaltic pump (14) to move the therapeutic substance from the drug container (12);
an orientation sensor (16) adapted to detect an orientation (V) of the drug container (12) relative to gravity (G); and
an operation processor (22) acquired a pre-set program, in response to delivered volumes of the therapeutic substance and the orientation (V) of the drug container (12), and adapted to provide an output signal to control delivery of the therapeutic substance.

2. The drug delivery device (10) of claim 1, **characterized in that** the drug container (12) is a vial made by hard materials, and the peristaltic pump (14) takes out the therapeutic substance from the vial by suction.

3. The drug delivery device (10) of claim 1, **characterized in that** the orientation sensor (16) acquires the orientation (V) of the drug container (12) by detecting an angle difference between a longitudinal direction (D) of the drug container (12) and a direction of the gravity (G).

4. The drug delivery device (10) of claim 1, **characterized in that** the operation processor (22) acquires the pre-set program containing specific parameters, comprises the delivered volumes and/or time progression of the delivery of the therapeutic substance.

5. The drug delivery device (10) of claim 4, **characterized in that** the operation processor (22) defines at least one of a first angle boundary (IAB) and a second angle boundary (OAB) in accordance with the specific parameters.

6. The drug delivery device (10) of claim 5, **characterized in that** the operation processor (22) acquires the pre-set program containing the specific parameters, the operation processor (22) pauses the delivery of the therapeutic substance when the orientation (V) of the drug container (12) is greater than the second angle boundary (OAB), the operation processor (22) maintains the delivery of the therapeutic substance when the orientation (V) of the drug container (12) is smaller than or equal to the second angle boundary (OAB), the operation processor (22) maintains the delivery of the therapeutic substance when the orientation (V) of the drug container (12) is smaller than or equal to the first angle boundary (IAB).

7. The drug delivery device (10) of claim 5, **characterized in that** the drug delivery device (10) further comprises a memory unit (20) electrically connected to the operation processor (22), the operation processor (22) acquires the specific parameters for searching the first angle boundary (IAB) and the second angle boundary (OAB) in accordance with the specific parameters from the memory unit (20).

8. The drug delivery device (10) of claim 1, **characterized in that** the drug delivery device (10) further comprises a case (24) or multiple cases (24) assembled together as a single structure adapted to accommodate the drug container (12), the peristaltic pump (14), the orientation sensor (16), the drive system (15), and the operation processor (22).

9. A drug delivery device (10), the drug delivery device (10) **characterized by**:
a drug container (12);
a peristaltic pump (14) engaged with the drug container (12) and adapted to deliver a therapeutic substance from the drug container (12) to a subject;
a drive system (15) adapted to drive the peristaltic pump (14) to move the therapeutic substance from the drug container (12);
an orientation sensor (16) adapted to detect an orientation (V) of the drug container (12) relative to gravity (G);
an operation processor (22) acquired a pre-set program, in response to delivered volumes of the therapeutic substance and the orientation (V) of the drug container (12), and adapted to provide an output signal to control delivery of the therapeutic substance; and
an alarm system (18), based on the output signal from the operation processor (22), and adapted to provide an interface output to inform users a delivery status of the therapeutic substance.

10. The drug delivery device (10) of claim 9, **characterized in that** the drug container (12) is a vial made by hard materials, and the peristaltic pump (14) takes out the therapeutic substance from the vial by suction.

11. The drug delivery device (10) of claim 9, **characterized in that** the orientation sensor (16) acquires the orientation (V) of the drug container (12) by detecting an angle difference between a longitudinal direction (D) of the drug container (12) and a direction of the gravity (G).

12. The drug delivery device (10) of claim 9, **characterized in that** the operation processor (22) acquired the pre-set program containing specific parameters, comprising the delivered volumes and/or time progression of the delivery of the therapeutic substance, the operation processor (22) defines at least one of a first angle boundary (IAB) and a second angle boundary (OAB) in accordance with the specific parameters.

13. The drug delivery device (10) of claim 12, **characterized in that** the alarm system (18) comprises a single or a plurality of alarm units, the operation processor (22) activates the alarm units to output an alarm signal or a combined alarm signal in response to the orientation (V) greater than the second angle boundary (OAB), and the operation processor (22) pauses the delivery of the therapeutic substance, the operation processor (22) activates the alarm units to output the alarm signal or the combined alarm signal in response to the orientation (V) greater than the first angle boundary (IAB) but smaller than or equal to the second angle boundary (OAB), and the operation processor (22) maintains the delivery of the therapeutic substance, the operation processor (22) is further adapted to maintain the delivery of the therapeutic substance and not actuate the alarm system (18) in response to the orientation (V) smaller than or equal to the first angle boundary (IAB).

14. The drug delivery device (10) of claim 12, **characterized in that** the drug delivery device (10) further comprises a memory unit (20) electrically connected to the operation processor (22), the operation processor (22) acquires the specific parameters for searching the first angle boundary (IAB) and the second angle boundary (OAB) in accordance with the specific parameters from the memory unit (20).

15. The drug delivery device (10) of claim 9, **characterized in that** the drug delivery device (10) further comprises a case (24) or multiple cases (24) assembled together as a single structure adapted to accommodate the drug container (12), the peristaltic pump (14), the orientation sensor (16), the drive system (15), and the operation processor (22), and the alarm system (18) is disposed inside the case (24) or on an outer surface of the case (24).

## Amended claims

### Amended claims in accordance with Rule 137(2) EPC.

1. A drug delivery device (10), the drug delivery device (10) **characterized by**:
a drug container (12);
a peristaltic pump (14) engaged with the drug container (12) and adapted to deliver a therapeutic substance from the drug container (12) to a subject;
a drive system (15) adapted to drive the peristaltic pump (14) to move the therapeutic substance from the drug container (12);
**characterized in that** the drug delivery device (10) further comprises:
an orientation sensor (16) adapted to detect an orientation (V) of the drug container (12) relative to gravity (G); and
an operation processor (22) adapted to acquire a pre-set program, in response to delivered volumes of the therapeutic substance for deciding at least one of angle boundaries to compare with the orientation (V) of the drug container (12), and adapted to provide an output signal to automatically control delivery of the therapeutic substance in accordance with a comparison result of the orientation (V) and the at least one of angle boundaries.

2. The drug delivery device (10) of claim 1, **characterized in that** the drug container (12) is a vial made by hard materials, and the peristaltic pump (14) takes out the therapeutic substance from the vial by suction.

3. The drug delivery device (10) of claim 1, **characterized in that** the orientation sensor (16) acquires the orientation (V) of the drug container (12) by detecting an angle difference between a longitudinal direction (D) of the drug container (12) and a direction of the gravity (G).

4. The drug delivery device (10) of claim 1, **characterized in that** the pre-set program acquired by the operation processor (22) contains specific parameters, the specific parameters comprises the delivered volumes and/or time progression of the delivery of the therapeutic substance.

5. The drug delivery device (10) of claim 4, **characterized in that** the operation processor (22) defines at least one of a first angle boundary (IAB) and a second angle boundary (OAB) in accordance with the specific parameters.

6. The drug delivery device (10) of claim 5, **characterized in that** the operation processor (22) acquires the pre-set program containing the specific parameters, the operation processor (22) pauses the delivery of the therapeutic substance when the orientation (V) of the drug container (12) is greater than the second angle boundary (OAB), the operation processor (22) maintains the delivery of the therapeutic substance when the orientation (V) of the drug container (12) is smaller than or equal to the second angle boundary (OAB), the operation processor (22) maintains the delivery of the therapeutic substance when the orientation (V) of the drug container (12) is smaller than or equal to the first angle boundary (IAB).

7. The drug delivery device (10) of claim 5, **characterized in that** the drug delivery device (10) further comprises a memory unit (20) electrically connected to the operation processor (22), the operation processor (22) acquires the specific parameters for searching the first angle boundary (IAB) and the second angle boundary (OAB) in accordance with the specific parameters from the memory unit (20).

8. The drug delivery device (10) of claim 1, **characterized in that** the drug delivery device (10) further comprises a case (24) or multiple cases (24) assembled together as a single structure adapted to accommodate the drug container (12), the peristaltic pump (14), the orientation sensor (16), the drive system (15), and the operation processor (22).

9. A drug delivery device (10), the drug delivery device (10) **characterized by**:
a drug container (12);
a peristaltic pump (14) engaged with the drug container (12) and adapted to deliver a therapeutic substance from the drug container (12) to a subject;
a drive system (15) adapted to drive the peristaltic pump (14) to move the therapeutic substance from the drug container (12);
**characterized in that** the drug delivery device (10) further comprises:
an orientation sensor (16) adapted to detect an orientation (V) of the drug container (12) relative to gravity (G);
an operation processor (22) adapted to acquire a pre-set program, in response to delivered volumes of the therapeutic substance for deciding at least one of angle boundaries to compare with the orientation (V) of the drug container (12), and adapted to provide an output signal to automatically control delivery of the therapeutic substance in accordance with a comparison result of the orientation (V) and the at least one of angle boundaries; and
an alarm system (18), based on the output signal from the operation processor (22), and adapted to provide an interface output to inform users a delivery status of the therapeutic substance.

10. The drug delivery device (10) of claim 9, **characterized in that** the drug container (12) is a vial made by hard materials, and the peristaltic pump (14) takes out the therapeutic substance from the vial by suction.

11. The drug delivery device (10) of claim 9, **characterized in that** the orientation sensor (16) acquires the orientation (V) of the drug container (12) by detecting an angle difference between a longitudinal direction (D) of the drug container (12) and a direction of the gravity (G).

12. The drug delivery device (10) of claim 9, **characterized in that** the pre-set program acquired by the operation processor (22) contains specific parameters, the specific parameters comprises the delivered volumes and/or time progression of the delivery of the therapeutic substance, the operation processor (22) defines at least one of a first angle boundary (IAB) and a second angle boundary (OAB) in accordance with the specific parameters.

13. The drug delivery device (10) of claim 12, **characterized in that** the alarm system (18) comprises a single or a plurality of alarm units, the operation processor (22) activates the alarm units to output an alarm signal or a combined alarm signal in response to the orientation (V) greater than the second angle boundary (OAB), and the operation processor (22) pauses the delivery of the therapeutic substance, the operation processor (22) activates the alarm units to output the alarm signal or the combined alarm signal in response to the orientation (V) greater than the first angle boundary (IAB) but smaller than or equal to the second angle boundary (OAB), and the operation processor (22) maintains the delivery of the therapeutic substance, the operation processor (22) is further adapted to maintain the delivery of the therapeutic substance and not actuate the alarm system (18) in response to the orientation (V) smaller than or equal to the first angle boundary (IAB).

14. The drug delivery device (10) of claim 12, **characterized in that** the drug delivery device (10) further comprises a memory unit (20) electrically connected to the operation processor (22), the operation processor (22) acquires the specific parameters for searching the first angle boundary (IAB) and the second angle boundary (OAB) in accordance with the specific parameters from the memory unit (20).

15. The drug delivery device (10) of claim 9, **characterized in that** the drug delivery device (10) further comprises a case (24) or multiple cases (24) assembled together as a single structure adapted to accommodate the drug container (12), the peristaltic pump (14), the orientation sensor (16), the drive system (15), and the operation processor (22), and the alarm system (18) is disposed inside the case (24) or on an outer surface of the case (24).
